(19) Europäisches Patentamt
European Patent Office
Office européen des brevets

(11) EP 1 173 430 B1

(12) **EUROPEAN PATENT SPECIFICATION**

(45) Date of publication and mention of the grant of the patent:
**09.07.2003 Bulletin 2003/28**

(21) Application number: **00917002.8**

(22) Date of filing: **28.03.2000**

(51) Int Cl.[7]: **C07D 307/33**

(86) International application number:
**PCT/EP00/02705**

(87) International publication number:
**WO 00/064882 (02.11.2000 Gazette 2000/44)**

(54) **ENANTIOMERS OF MERCAPTO LACTONES AND PROCESSES FOR THEIR SYNTHESIS**

ENANTIOMERE VON MERCAPTOLACTONEN UND VERFAHREN ZU IHRER HERSTELLUNG

ENANTIOMERES DE MERCAPTOLACTONES ET LEURS PROCEDES DE SYNTHESE

(84) Designated Contracting States:
**AT BE CH CY DE DK ES FI FR GB GR IE IT LI LU MC NL PT SE**

(30) Priority: **23.04.1999 GB 9909229**

(43) Date of publication of application:
**23.01.2002 Bulletin 2002/04**

(73) Proprietor: **GLAXO GROUP LIMITED**
**Greenford, Middlesex UB6 ONN (GB)**

(72) Inventors:
- **ANDERSEN, Marc, Werner**
  **Raleigh, NC 27612 (US)**
- **BOSWELL, Grady, Evan**
  **Fountain Inn, SC 29644 (US)**
- **SHARP, Matthew, Jude, Glaxo Wellcome Inc.**
  **Research Triangle Park, NC 27709 (US)**
- **ZHOU, Xiaoming, Glaxo Wellcome Inc**
  **Research Triangle Park, NC 27709 (US)**

(74) Representative: **Giddings, Peter John, Dr. et al**
**GlaxoSmithKline**
**Corporate Intellectual Property (CN9.25.1)**
**980 Great West Road**
**Brentford, Middlesex TW8 9GS (GB)**

(56) References cited:
**WO-A-97/24365**

- **CHEMICAL ABSTRACTS, vol. 66, no. 7, 13 February 1967 (1967-02-13) Columbus, Ohio, US; abstract no. 28363, FUCHS, GEORG: "Some 2- or 3-mercapto substituted.gamma.-lactones" XP002152698 cited in the application & ARK. KEMI (1966), 26(10), 111-16 ,**
- **ZHENG T -C ET AL: "A General and Mild Synthesis of Thioesters and Thiols from Halides" TETRAHEDRON LETTERS,NL,ELSEVIER SCIENCE PUBLISHERS, AMSTERDAM, vol. 40, no. 4, 22 January 1999 (1999-01-22), pages 603-606, XP004151396 ISSN: 0040-4039**
- **SH.-JAN SHIUEY ET AL.: "Triply Convergent Synthesis of 1alpha,25-Dihydroxy-24(R)-fluorocholecalciferol" JOURNAL OF ORGANIC CHEMISTRY, vol. 53, 1988, pages 1040-1046, XP002152696 EASTON US**
- **F. MATSUDA ET AL.: "A Formal Synthesis of Aplasmomycin" CHEMISTRY LETTERS, 1987, pages 2097-2100, XP002152697 TOKYO JP**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 173 430 B1

## Description

The present invention relates to a new process for the synthesis of enantiomers of mercapto lactones, and their acyl derivatives which are useful intermediates in the preparation of compounds of formula (I).

Compounds of formula (I) have the following structure

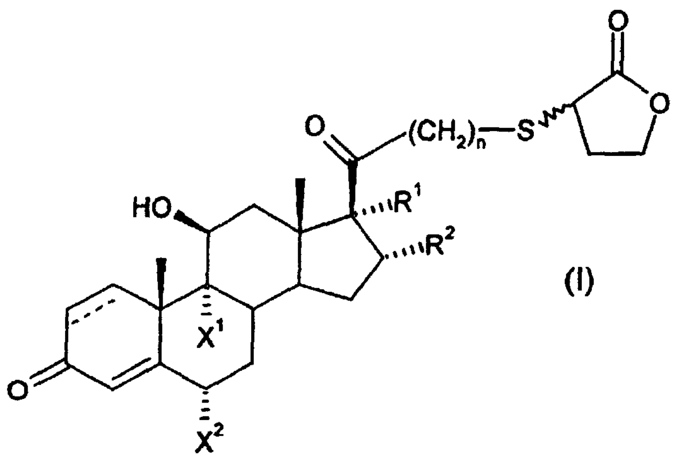

(I)

in which $R^1$ individually represents $OC(=O)C_{1-6}alkyl$; $R^2$ individually represents hydrogen, methyl (which may be in either the $\alpha$ or $\beta$ configuration) or methylene; or $R^1$ and $R^2$ together represent

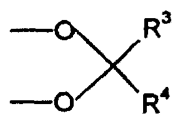

wherein $R^3$ and $R^4$ are the same or different and each represents hydrogen or $C_{1-6}$ alkyl; $X^1$ and $X^2$ are the same or different and each represents hydrogen or halogen; $\overline{----}$ represents a single or double bond; and n=0 or 1. These compounds were first described in PCT patent publications WO97/24365 and WO97/24367. Currently there is considerable interest in compounds of formula (I), for example compound A, as antiinflammatory and anti-allergic compounds in the treatment of asthma and other inflammatory diseases.

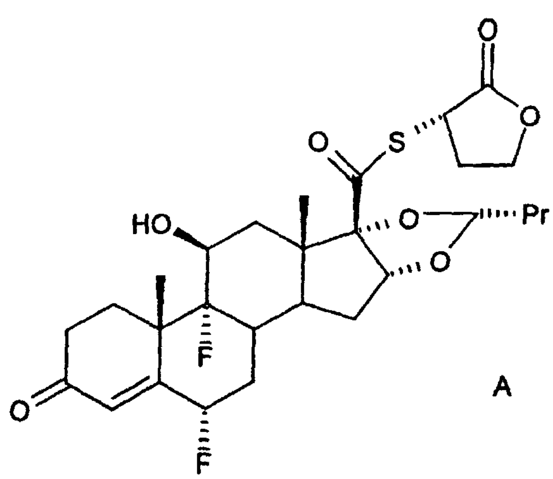

A

Compound A is 16$\alpha$, 17$\alpha$-(R-butylidenedioxy)-6$\alpha$,9$\alpha$-difluoro-11$\beta$-hydroxy-3-oxo-androst-4-ene-17$\beta$-carbothioic acid *S*-(2-oxo-tetrahydro-furan-3S-yl) ester.

The route of synthesis of the compound A described in the specification of WO97/24365 is specifically described therein in example 19.

One route to compounds of general formula (I), in which n = 0, described in WO97/24365 involves coupling a carboxylic acid of formula (II)

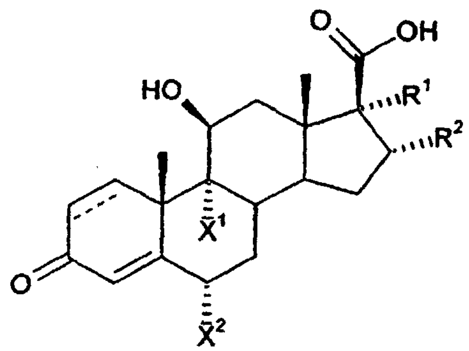

(II)

with racemic $\alpha$-mercapto-$\gamma$-butyrolactone (formula (III))

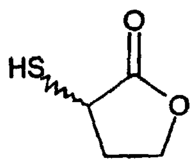

(III)

to yield a 1:1 mixture of lactone diastereoisomers of formula (I) which require separation. Due to the separation step this method is not amenable to large scale synthesis.

A further route referred to in WO97/24365 for when n=0 involves the conversion of a compound of formula (II) to a compound of formula (IV), using difficult and toxic reagents such as hydrogen sulphide gas.

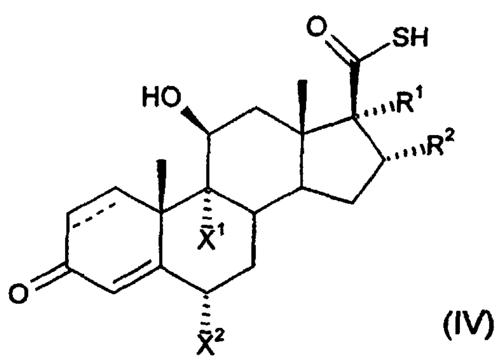

(IV)

The compound of formula (IV) is then reacted with an enantiomerically pure compound of formula (V)

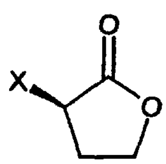

(V)

in which X represents a suitable leaving group, (for example chloro, bromo, or $OSO_2A$ wherein A is $CH_3$, $CF_3$, or p-$CH_3C_6H_4$) to yield a single diastereoisomer of formula (I). This process requires the use of hydrogen sulphide gas, which is very toxic and difficult to handle. Furthermore, the compound of formula (IV) is not stable on storage and

must be kept cold. Also, the route is long since it requires an additional synthetic transformation.

One route to compounds of general formula (I), in which n = 1, described in WO97/24365, involves treating a compound of formula (VI)

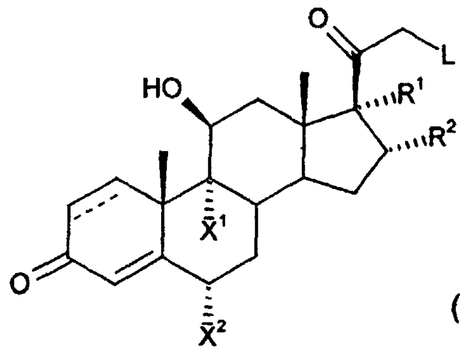

(VI)

wherein $R^1$, $R^2$, $X^1$ and $X^2$ are as previously defined, and L represents a suitable leaving group such as a sulphonate ester, for example a mesylate, tosylate or triflate, or L represents a halogen such as chloro or bromo, with racemic a-mercapto-y-butyrolactone (formula (III))

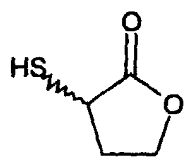

(III)

to yield a 1:1 mixture of lactone diastereoisomers of formula (I) wherein n = 1 which require separation. Due to the separation step this method is not amenable to large scale synthesis.

An ideal route for the synthesis of a compound of formula (I) should produce the compound in high yields at a reasonable speed and at low cost with minimum waste materials and in a manner that is of minimum impact to the environment in terms of energy consumption and disposal of waste-materials.

We have found a new process for the synthesis of the enantiomers of compounds of formula (III), providing many advantages in the preparation of compounds of formula (I) over previously known routes of synthesis. Such advantages include avoiding the need for a difficult separation step, avoiding the use of unpleasant and toxic reagents such as hydrogen sulphide gas, lower cost, less waste, and more efficient use of materials.

The single enantiomers of compound (III), (S)-α-mercapto-γ-butyrolactone and (R)-α-mercapto-γ-butyrolactone, are represented by the formulae (IIIa) and (IIIb).

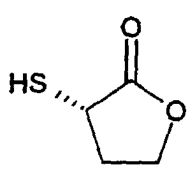

(IIIa)

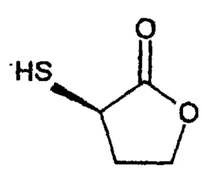

(IIIb)

The single enantiomer of formula (IIIa), substantially free of the corresponding enantiomer, may be prepared by the following reaction sequence, which is presented as a feature of the present invention:

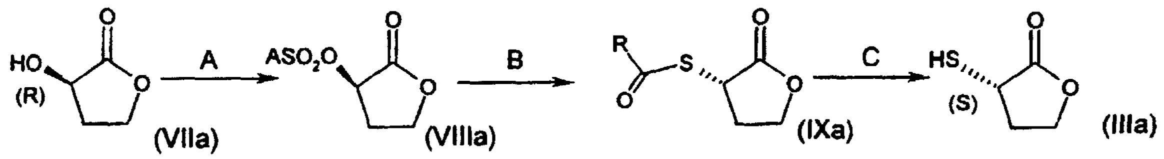

(VIIa) (VIIIa) (IXa) (IIIa)

In formula (VIIIa), A represents $CH_3$, $CF_3$, or p-$CH_3C_6H_4$, more preferably $CH_3$. In formula (IXa), R represents $C_{1-6}$ alkyl or phenyl optionally substituted by one or more substituents selected from halo, $C_{1-3}$alkyl, hydroxy, and $C_{1-3}$ alkoxy, more preferably R is $CH_3$.

Step A is carried out by treating a compound of formula (VIIa) with a sulfonyl chloride or sulfonic anhydride, preferably methanesulfonyl chloride or trifluoromethanesulfonyl chloride (most preferably methanesulfonyl chloride) preferably in the presence of a base, for example a trialkylamine base such as triethylamine or diisopropylethylamine, or an aromatic base such as pyridine, lutidine or 4-N,N-dimethylaminopyridine (most preferably the base will be triethylamine or 4-N,N-dimethylaminopyridine, especially triethylamine and 4-N,N-dimethylaminopyridine), and preferably in a suitable solvent, for example aromatic solvents such as toluene, ester solvents such as ethyl acetate, ether solvents such as tetrahydrofuran (THF), dimethoxyethane and dioxane, amides such as dimethylformamide, ketones such as methyl isobutyl ketone, or chlorinated hydrocarbons such as dichloromethane. Preferably the solvent is selected from the group consisting of dichloromethane, ethyl acetate and methyl isobutyl ketone; most preferably the solvent is ethyl acetate. Preferably, the reaction is carried out at a temperature in the range of -40°C to ambient temperature, most preferably from 10°C to 20°C. Step A will then be completed by recrystallisation of the compound of formula (VIIIa) according to appropriate conditions, preferably using ethyl acetate for recrystallisation.

Step B is carried out by treating a compound of formula (Villa) with a thiocarboxylate salt (especially a thioacetate salt). Suitable thiocarboxylate salts include inorganic salts (such as potassium or cesium), trialkylamine salts (such as triethylamine), or aromatic amine salts (such as pyridine and lutidine). The thiocarboxylate salts are either commercially available, prepared by standard methods, or generated in situ from thiocarboxylic acid; the preferred thiocarboxylate salt is potassium thioacetate. Preferably, the reaction is carried out in a suitable solvent, for example aromatic solvents such as toluene, esters such as ethyl acetate, amides such as dimethylformamide, ethers such as THF, dioxane, and dimethoxyethane, or chlorinated hydrocarbons such as dichloromethane. Preferably the solvent is selected from the group consisting of toluene, dimethoxyethane and THF; more preferably, the solvent is toluene. Preferably, the reaction is carried out at a temperature in the range of -20°C to ambient temperature, most preferably room temperature (20°C).

Step C is carried out by reacting a compound of formula (IXa) with an aliphatic alcohol, for example methanol or ethanol, preferably methanol, in the presence of an inorganic acid, for example hydrochloric acid, or alternatively with an aromatic amine, for example 4-chloroaniline; preferably the aliphatic alcohol will be present with hydrochloric acid. Preferably the reaction is carried out in the presence of a suitable solvent, for example aromatic solvents such as toluene, ether solvents such as dioxane or THF, or chlorinated solvents such as dichloromethane. Preferably, the solvent is toluene or dioxane; more preferably, the solvent is toluene. Step C will then be completed by vacuum distillation of the compound of formula (IIIa) according to appropriate conditions.

The single enantiomer of formula (IIIb), substantially free of the corresponding enantiomer, may be prepared by an analogous process in which reagents and conditions are as in steps A, B and C above, and which is presented as a further feature of the present invention, using as starting material the (S) enantiomer (VIIb) as follows

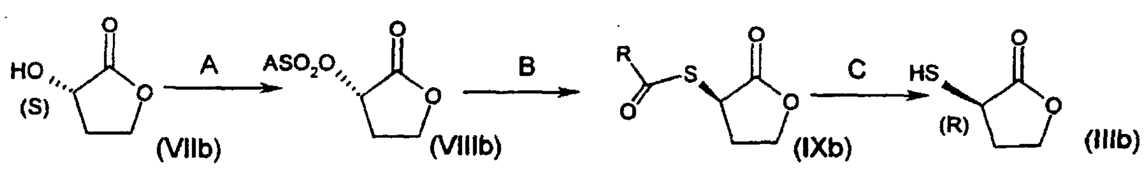

(VIIb) (VIIIb) (IXb) (IIIb)

Substituents A and R in formulae (VIIIb) and (IXb) respectively are as defined above for formulae (VIIIa) and (IXa).

Enantiomeric compounds (IIIa), (IIIb), (IXa) and (IXb), substantially free of the corresponding enantiomer, are novel and are presented as further features of the present invention.

By 'substantially free' is meant no more than about 10% w/w of the corresponding enantiomer, particularly no

more than about 5% w/w, and more particularly less than about 3% w/w is present.

Compounds (IXa) and (IXb) wherein R is methyl, are (S)-α-acetylthio-γ-butyrolactone and (R)-α-acetylthio-γ-butyrolactone respectively.

Alternatively, the single enantiomers (IXa) and (IIIb) may be prepared by an enzymatic transacetylation reaction of racemic compound (IX), which is presented as a further feature of the present invention. Compound (IXa) prepared in this manner may then be separated from compound (IIIb) by distillation and converted to compound (IIIa) as illustrated in the reaction scheme above.

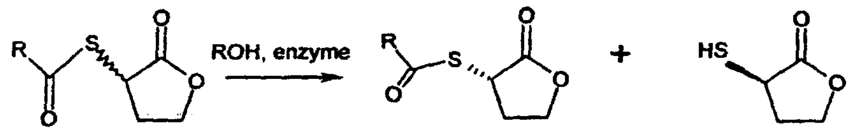


Racemic (IX) (IXa) (IIIb)

This transacetylation reaction is preferably carried out in the presence of an alcohol, such as butanol, benzyl alcohol, hexanol or octanol, most preferably butanol, and a suitable lipase or esterase enzyme, preferably *mucor meihei* lipase, in toluene and/or isooctane, most preferably toluene, containing a small amount of water. Preferably, the reaction will be carried out at a temperature in the range of -20°C to room temperature, most preferably 5°C.

The compounds (VIIa) and (VIIb) are commercially available. Racemic compound (IX) may be prepared by the method described in Fuchs, Swed. Ark. Kemi (1966), 26 (10), 111-116. Compounds of formula (II) may be prepared by methods known in the art, for example by the methodology described in Kertesz and Marx, Journal of Organic Chemistry, 1986, 51, 2315-2328, the contents of which are incorporated herein by reference hereto.

Compounds (IIIa) and (IIIb) may be used directly in the preparation of single diastereoisomers of compounds of formula (I) in which n=0 by reaction with a compound of formula (II):

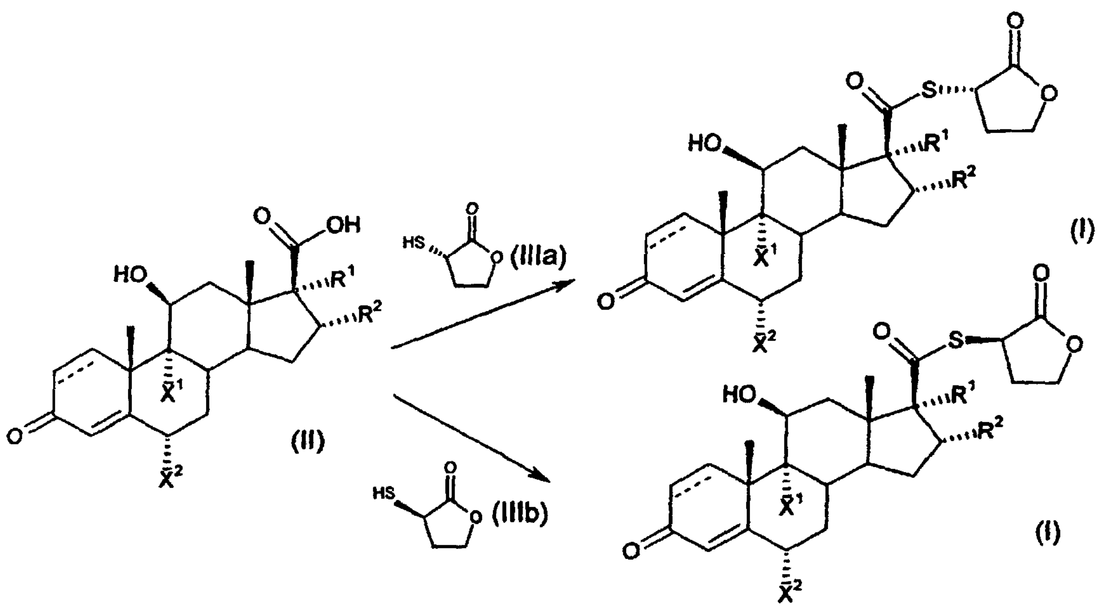

(II) (IIIa) (IIIb) (I) (I)

This represents a cost effective, convergent synthesis of a single diastereoisomer of formula (I) in high yield with no separation step.

This reaction is presented as a further feature of the present invention. Preferably, the reaction is carried out in a solvent, for example amide solvents such as N,N-dimethylformamide and N-methyl-2-pyrrolidinone; ester solvents such as ethyl acetate and isopropyl acetate; ketone solvents such as acetone, 2-butanone and 4-methyl-2-pentanone; hydrocarbon solvents such as toluene; halogenated solvents such as dichloromethane; and nitrile solvents such as acetonitrile. Preferably, the solvent is acetone.

The reaction is carried out in the presence of a base. Preferably the base is a weak inorganic base such as sodium acetate or sodium bicarbonate. Potassium carbonate is an alternative base, but sodium bicarbonate is especially preferred.

The reaction is carried out in the presence of a coupling agent. Preferably, the coupling agent is *O*-benzotriazole-1-yl-*N,N,N',N'*-tetramethyluronium hexafluorophosphate (or its corresponding tetrafluoroborate salt). Preferably, the coupling agent is added to the compound of formula (II) at a temperature in the range 0 to 100 °C. Preferably, the coupling step with the compound of formula (III) is carried out at a temperature in the range -10 °C to ambient temperature, most preferably 4°C. This reaction will then be completed by recrystallisation of the compound of formula (I) according to appropriate conditions, preferably using isopropyl acetate and heptane for recrystallisation.

Compounds of formula (II) may alternatively be prepared according to the following process:

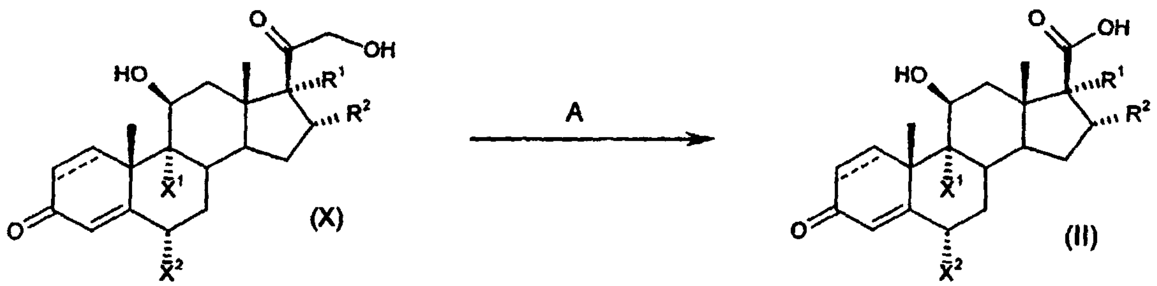


(X) (II)

Step A is carried out in the presence of hydrogen peroxide and a base. Preferably the base is a weak inorganic base such as potassium carbonate, preferably the reaction will be carried out in the presence of an aliphatic alcohol, most preferably methanol, preferably at room temperature. Step A will then be completed by recrystallisation of the compound of formula (II) according to appropriate conditions, preferably using ethyl acetate for recrystallisation.

Compounds (IIIa) and (IIIb) may also be used directly in the preparation of single diastereoisomers of compounds of formula (I) in which n=1 by reaction with a compound of formula (VI):

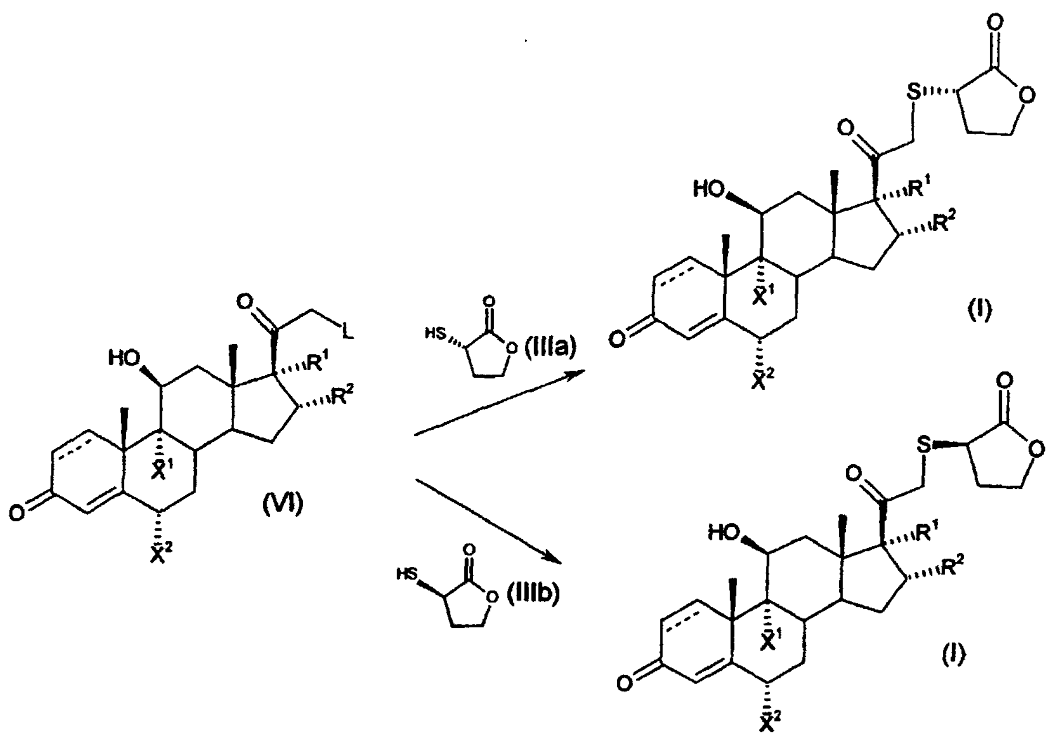

(VI) (IIIa) (IIIb) (I) (I)

This represents a cost effective, convergent synthesis of a single diastereoisomer of formula (I) where n = 1 in high yield with no separation step. The reaction conditions are as described above for the reaction of compounds of formulae (II) and (IIIa) or (IIIb).

The invention is further described by following examples, which are presented by way of illustration only.

In the Examples, unless otherwise stated:

All temperatures refer to °C. Proton Magnetic Resonance ($^1$H-NMR) spectra were recorded at 300 MHz, chemical shifts are reported in ppm (δ) from $Me_4Si$, used as internal standard , and are assigned as singlets (s), doublets (d), doublet of doublets (dd), triplets (t), doublet of triplets (dt), quartets (q) or multiplets (m). The following abbreviations are used in text: AcOEt = ethyl acetate, TFA = trifluoroacetic acid, HCl = hydrochloric acid, EtOH = ethanol.

## Example 1

(S)-α-mercapto-γ-butyrolactone

A. (R)-α-(Methylsulfonyl)oxy-γ-butyrolactone

Triethylamine (45ml, 323mmol) and 4-N,N-dimethylaminopyridine (3.6g, 29mmol) in anhydrous AcOEt (75ml) were added to a stirred solution of (R)-(+)-α-hydroxy-γ-butyrolactone (30g, 294mmol) in anhydrous AcOEt (300ml), under $N_2$, at -40°. The solution was stirred for 10min. Methanesulfonyl chloride (25ml, 323mmol) was added slowly to the reaction, maintaining the temperature below -35°. The mixture was stirred for 2h at -40°. Upon completion the mixture was allowed to warm up to ambient temperature. The mixture was diluted with AcOEt (450ml) and filtered. The solid was washed with AcOEt (3x60ml). The filtrate was washed with a solution of 50% $H_2O$ and 50% brine (60ml). The organic layer was washed with 1 N HCl (45ml), a solution of 50% $H_2O$ and 50% saturated brine (45ml), a saturated aqueous $NaHCO_3$ solution (45ml), and a solution of 50% $H_2O$ and 50% brine (2x45ml). The solvent (780ml) was removed under reduced pressure and hexanes (500 ml) was added to the mixture. The mixture was stirred at 5° for 2h. The mixture was filtered, and solid was dried under vacuum at room temperature to yield (R)-α-(methylsulfonyl) oxy-γ-butyrolactone as a white solid (45g, 85%). $^1$H-NMR ($CDCl_3$): δ 5.32 (1H, t, J=8.7Hz), 4.52 (1H, m), 4.33 (1H, m), 3.27 (3H, s), 2.76 (1H, m), 2.56 (1H, m).

B. (S)-α-acetylthio-γ-butyrolactone

Potassium thioacetate (7.33g, 64.17mmol) was added to a solution of (R)-α-(methylsulfonyl)oxy-γ-butyrolactone (11g, 61.11mmol) in toluene (220ml) at 5°. The reaction mixture was stirred at ambient temperature for 36 h. The mixture was filtered and the solid was washed with toluene (3x22ml). The organic layer was washed with water (3 ml) and 1N HCl (3 ml). The organic solution was dried over sodium sulfate, the toluene was removed by rotary evaporation to give (S)-α-acetylthio-γ-butyrolactone as a liquid (9.2g, 94% yield): 98% e.e. (HPLC, column: Daicel AD, 10% EtOH / Hexanes, flow 1ml/min); $^1$H-NMR ($CDCl_3$), δ 4.40 (1H, m), 4.26 (1H, m), 4.23 (1H, m), 2.70 (1H, m), 2.35 (3H, s), 2.23 (1H, m). $^{13}$C-NMR (75MHz, $CDCl_3$): δ 193.59, 174.72, 66.95, 40.46, 30.42, 30.30. Anal. Calcd for $C_6H_8O_3S$ : C, 44.99; H, 5.03; S, 20.02. Found: C, 44.81; H, 5.05; S, 20.10%.

C. (S)-α-mercapto-γ-butyrolactone

Methanol (46ml) and 4M HCl in dioxane (9.2ml, 36.8mmol) were added to (S)-α-acetylthio-γ-butyrolactone (9.2g, 58mmol) in toluene (138ml) at 0°. The solution was stirred at ambient temperature overnight. Upon completion of the reaction, 92ml of solvents were removed under vacuum. Toluene (92ml) and TFA *(0.1* ml) was added to give a pH of 1.5-2.0. The solution was stirred overnight. Toluene was removed under vacuum to give crude product. The crude product was dried under vacuum for 15min at ambient temperature. Vacuum distillation gave (S)-α-mercapto-γ-butyrolactone (5.9g, 87% yield) (b.p. 50-51°, 200millitorr): 98% e.e. (HPLC, column: Daicel AD, 10% EtOH/Hexanes, flow 1ml/min). $^1$H-NMR ($CDCl_3$): δ 4.47 (1H, m), 4.32 (1H, m), 3.72 (1H, m), 2.69 (1H, m), 2.33 (1H, d, J=4.8Hz), 2.18 (1H, m). $^{13}$C-NMR (75MHz, $CDCl_3$): δ 176.89, 66.77, 34.97, 32.09. Anal. Calcd for $C_4H_6O_2S$ : C, 40.66; H, 5.12; S, 27.14. Found: C, 40.61; H, 5.13; S, 26.99%.

**Example 2**

16α, 17α-(R-butylidenedioxy)-6α,9α-difluoro-11β-hydroxy-3-oxo-androst-4-ene-17β-carbothioic acid S-(2-oxo-tetrahydro-furan-3S-yl) ester.

Step A)

6α, 9α, Difluoro-11β-hydroxy-16α, 17α-isopropylidenedioxy-3-oxo-androst-4-ene-17β carboxylic acid

A mixture of 1,2-dihydrofluocinolone acetonide (5.0g, 11.0 mmol), potassium carbonate (4.56g, 33.0 mmol) and hydrogen peroxide (18.5 ml, 16.5 mmol as a 3% wt/vol. solution in water)was stirred in methanol (35 ml) at ambient temperature for 24 hours. The mixture was then treated with additional hydrogen peroxide (12 ml, 11.0 mmol ) for an additional 24-hour period. Water (25 ml) was added and the solution was washed with ethyl acetate (25 ml) followed by hexanes (25 ml). The aqueous layer was then adjusted to pH 2 by addition of concentrated HCl, which resulted in a precipitate. The acidic aqueous layer was washed with ethyl acetate (2x25 ml) and the combined ethyl acetate extracts evaporated to dryness. The resulting off white solid was triturated in 1:1 hexanes-ethyl acetate (30ml) and filtered and dried at 50°C for 24 hours to provide the title compound as a off-white solid. (3.93g, 81%). 1H-NMR (DMSO-d6): 5.77(s,1H), 5.55 and 5.40(2m, 1H), 5.1(m,1H), 4.90(m,1H), 4.10(m,1H), 2.5-2.35(m, 3H), 2.35-2.1(2m, 3H), 2.0-1.85(m, 3H), 1.7-1.4 (m, overlapping s at 1.45, 5H) 1.35-1.20(m overlapping s at 1.29, 4H), 1.13 (s, 3H), 0.9 to 0.75 (m, overlapping s at 0.87, 4H).

Step B)

16α,17α-(R-butylidenedioxy)-6α,9α-difluoro-11β-hydroxy-3-oxo-androst-4-ene-17β-carboxylic acid

70% perchloric acid (22.8ml, 264mmol) was added to a stirred mixture butyraldehyde (11ml, 122mmol) and 6α,9α-difluoro-11β-hydroxy-16α,17α-isopropylidenedioxy-3-oxo-androst-4-ene-17β carboxylic acid (30.40g, 69mmol) in dichloromethane (608ml) at -3 °C. The mixture was stirred overnight at -3 °C and then 6% aqueous sodium carbonate ( 248ml) was added. The mixture was then allowed to warm to room temperature and 6% aqueous sodium carbonate (304ml) was added. After 3 hours the phases were separated and the organic phase was extracted with 6% aqueous sodium carbonate ( 2x60ml ). The combined aqueous phase was washed with toluene (2x150ml). Then, the aqueous phase was rapidly stirred as conc. hydrochloric acid was added until a pH of 2-1 was obtained. The resulting slurry was suction-filtered, and the filter plug was washed with water (3x60ml) and dried in air overnight to give a crude product (31g, containing 9% of the undesired acetal epimer). The crude product was dissolved in EtOH-water (1:2, ca. 532ml) by heating at 72-73 °C and the resulting solution was allowed to cool to room temperature overnight. The mixture was filtered, and the solid was washed with 30ml of water. The solid was dried in a vacuum oven at 90-100 °C to give the product (24g) with 1.6% of epimer. Recrystallization was repeated (12vol EtOH-water 1:2, ca. 430 ml). The product was dried in a vacuum oven at 90-100 °C to provide the title compound as an off-white solid (21.6g, 69% total yield, containing 0.3-0.5 % of the undesired epimer). $^1$H-NMR (300 MHz, DMSO, $d_6$) δ 0.86 (t, 3H); 0.92 (s, 3H); 1.31-1.57 (m, 7H); 1.47 (s, 3H); 1.74 (d, 1H); 1.91-2.42 (m, 8H); 4.14 (m, 1H); 4.62 (t, 1H); 4.87 (br, 1H); 5.14 (br, 1H); 5.42-5.57(m, 1H); 5.81 (s, 1H).

Step C)

16α,17α-(R-butylidenedioxy)-6α,9α-difluoro-11β-hydroxy-3-oxo-androst-4-ene-17β-carbothioic acid *S*-(2-oxo-tetrahydro-furan-3S-yl) ester.

A mixture of 16α,17α-(R-butylidenedioxy)-6α,9α-difluoro-11β-hydroxy-3-oxo-androst-4-ene-17β-carboxylic acid (6.00g, 13.2mmol), *O*-benzotriazole-1-yl-*N,N,N',N'*-tetramethyluronium hexafluorophosphate (HBTU, 5.98g, 15.8mmol), and sodium bicarbonate (2.28g, 27mmol), in acetone (45ml) was stirred under nitrogen and heated at 43° for 16h. The mixture was cooled to 0° and treated with more sodium bicarbonate (2.28g, 27mmol) then a solution of (S)-α-mercapto-γ-butyrolactone (ca. 98% e.e., 2.34g, 19.80mmol) in acetone (3ml). The mixture was stirred at 0° for 25h and the cold reaction mixture was then poured into isopropyl acetate (100ml). 1 N HCl (50 ml) was then added and the layers were separated. The organic layer was washed with 5% potassium carbonate (50 ml) then brine (50 ml) and dried over sodium sulfate, filtered, and concentrated under reduced pressure. The residue was then crystallized from hot isopropylacetate (24ml). The resulting slurry was suction-filtered and the white solid dried in a vacuum oven at 80° for 3 days affording the title compound (5.45g, 75% yield). M.pt. 169-173°; $^1$H-NMR ($CDCl_3$) δ 6.13 (1H,s); 5.26 (1H, dm, J = 47.5 Hz); 4.84 (1H, d, J = 5.2 Hz); 4.73 (1H, t, J = 4.2Hz); 4.47 (1H, dt, J = 8.6 Hz, 2.9 Hz); 4.4 - 4.28 (3H,m); 2.75 (1H,m); 2.6 - 2.4 (3H,m); 2.4 - 2.18 (4H,m); 2.0 (1H,m); 1.9 - 1.6 (8H,m); 1.52 (3H,s); 1.46 (2H, q, J = 7 Hz); 1.00 (3H,s); 0.95 (3H, t, J = 7.3 Hz).

The compound of Example 2 is capable of preparation according to the invention at an overall yield of 30% in 4 linear steps from the starting material (fluocinoline acetonide), resulting in a product with <2% of the undesired lactone isomer. This is clearly advantageous when compared with previously known syntheses, the best of which obtain the compound in 5 linear steps from identical starting material, obtaining an overall yield of 8% and resulting in a much less pure end product (approximately 5% of the undesired lactone isomer).

## Claims

1. (S)-α-mercapto-γ-butyrolactone containing no more than 10% w/w of the corresponding enantiomer.

2. (S)-α-mercapto-γ-butyrolactone containing no more than 5% w/w of the corresponding enantiomer.

3. (S)-α-mercapto-γ-butyrolactone containing less than 3% w/w of the corresponding enantiomer.

4. (R)-α-mercapto-γ-butyrolactone containing no more than 10% w/w of the corresponding enantiomer.

5. (R)-α-mercapto-γ-butyrolactone containing no more than 5% w/w of the corresponding enantiomer.

6. (R)-α-mercapto-γ-butyrolactone containing less than 3% w/w of the corresponding enantiomer.

7. A process for the preparation of a compound of formula (IIIa)

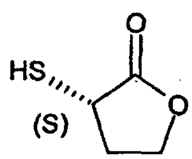

(IIIa)

comprising reacting a compound of formula (IXa)

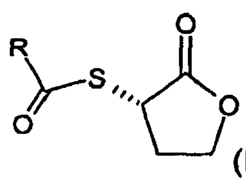

(IXa)

wherein R represents $C_{1-6}$ alkyl or phenyl optionally substituted by one or more substituents selected from halo, $C_{1-3}$alkyl, hydroxy, and $C_{1-3}$ alkoxy; with an aliphatic alcohol in the presence of hydrochloric acid.

8. A process for the preparation of a compound of formula (IIIb)

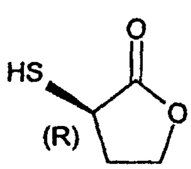

(IIIb)

comprising reacting a compound of formula (IXb)

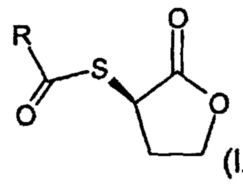

(IXb)

wherein R represents $C_{1-6}$ alkyl or phenyl optionally substituted by one or more substituents selected from halo, $C_{1-3}$alkyl, hydroxy, and $C_{1-3}$ alkoxy; with an aliphatic alcohol in the presence of hydrochloric acid.

**9.** A process for the preparation of compounds of formulae (IXa) and (IIIb)

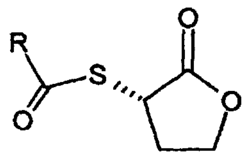

+

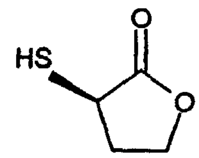

(IXa) (IIIb)

wherein R represents $C_{1-6}$ alkyl or phenyl optionally substituted by one or more substituents selected from halo, $C_{1-3}$alkyl, hydroxy, and $C_{1-3}$ alkoxy; comprising the enzymatic transacetylation of racemic compound (IX)

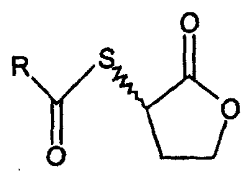

Racemic (IX)

wherein R is as defined above, in the presence of an alcohol and a suitable lipase or esterase enzyme in a solvent containing water.

**10.** A process for the preparation of a single enantiomer of a compound of formula (I)

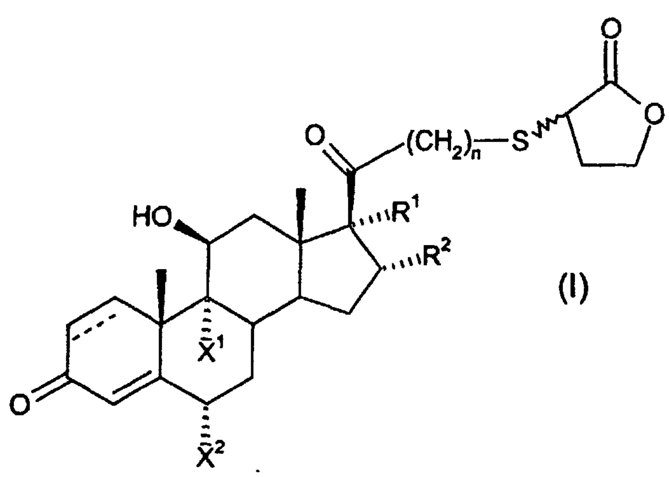

(I)

in which $R^1$ individually represents $OC(=O)C_{1-6}$alkyl; $R^2$ individually represents hydrogen, methyl (which may be in either the α or β configuration) or methylene; or $R^1$ and $R^2$ together represent

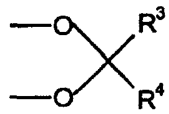

wherein $R^3$ and $R^4$ are the same or different and each represents hydrogen or $C_{1-6}$ alkyl; $X^1$ and $X^2$ are the same or different and each represents hydrogen or halogen; ---- represents a single or double bond; and n=0; comprising the reaction of a compound of formula (IIIa) or (IIIb)

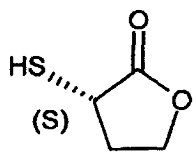 (IIIa) 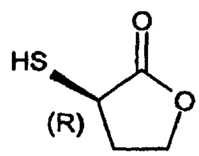 (IIIb)

with a compound of formula (II)

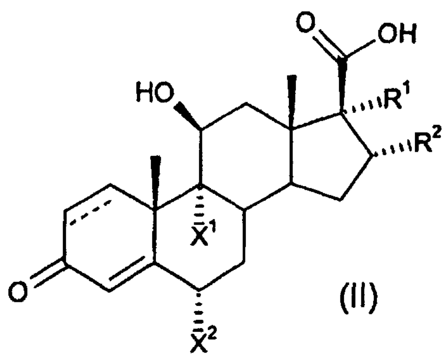

(II)

in which $R^1$, $R^2$, $X^1$ and $X^2$ are as defined above for formula (I), in the presence of a base and a coupling agent.

**11.** A process according to claim 10 wherein the base is sodium bicarbonate and the coupling agent is *O*-benzotriazole-1-yl-*N,N,N',N'*-tetramethyluronium hexafluorophosphate.

**12.** A process for the preparation of a single enantiomer of a compound of formula (I)

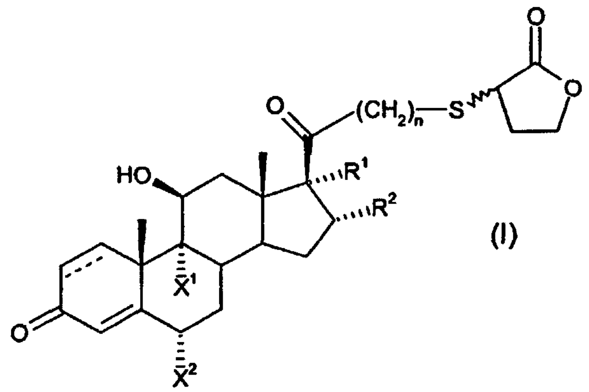

(I)

in which $R^1$ individually represents $OC(=O)C_{1-6}$alkyl; $R^2$ individually represents hydrogen, methyl (which may be in either the α or β configuration) or methylene; or $R^1$ and $R^2$ together represent

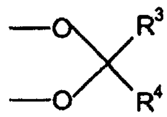

wherein $R^3$ and $R^4$ are the same or different and each represents hydrogen or $C_{1-6}$ alkyl; $X^1$ and $X^2$ are the same or different and each represents hydrogen or halogen; ---- represents a single or double bond; and n=1; comprising the reaction of a compound of formula (IIIa) or (IIIb)

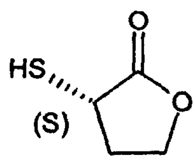

(IIIa)

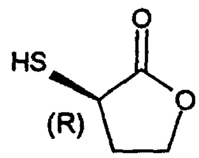

(IIIb)

with a compound of formula (VI)

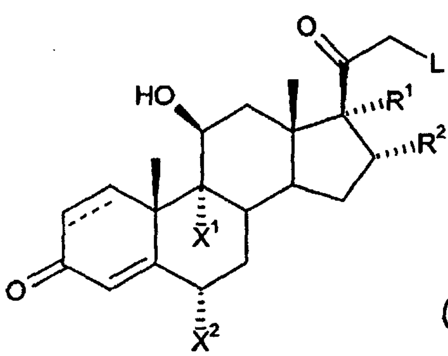

(VI)

in which $R^1$, $R^2$, $X^1$ and $X^2$ are as defined above for formula (I), and L represents a suitable leaving group, in the presence of a base and a coupling agent.

**13.** A process according to claim 12 wherein the base is sodium bicarbonate and the coupling agent is *O*-benzotriazole-1-yl-*N,N,N',N'*-tetramethyluronium hexafluorophosphate.

**Patentansprüche**

1. (S)-α-Mercapto-γ-butyrolacton, das nicht mehr als 10 % G/G des korrespondierenden Enantiomers enthält.

2. (S)-α-Mercapto-γ-butyrolacton, das nicht mehr als 5 % G/G des korrespondierenden Enantiomers enthält.

3. (S)-α-Mercapto-γ-butyrolacton, das weniger als 3 % G/G des korrespondierenden Enantiomers enthält.

4. (R)-α-Mercapto-γ-butyrolacton, das nicht mehr als 10 % G/G des korrespondierenden Enantiomers enthält.

5. (R)-a-Mercapto-γ-butyrolacton, das nicht mehr als 5 % G/G des korrespondierenden Enantiomers enthält.

6. (R)-α-Mercapto-γ-butyrolacton, das weniger als 3 % G/G des korrespondierenden Enantiomers enthält.

7. Verfahren zur Herstellung einer Verbindung der Formel (IIIa)

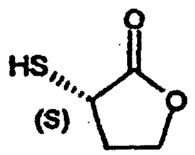

(IIIa)

umfassend das Umsetzen einer Verbindung der Formel (IXa):

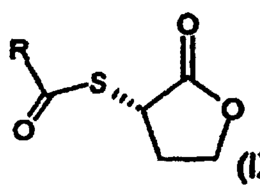

(IXa)

worin R $C_{1-6}$-Alkyl oder Phenyl darstellt, gegebenenfalls substituiert mit einem oder mehreren Substituenten, die aus Halogen, $C_{1-3}$-Alkyl, Hydroxy und $C_{1-3}$-Alkoxy ausgewählt sind; mit einem aliphatischen Alkohol in Gegenwart von Salzsäure.

8. Verfahren zur Herstellung einer Verbindung der Formel (IIIb):

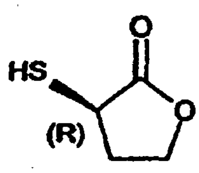

(IIIb)

umfassend das Umsetzen einer Verbindung der Formel (IXb):

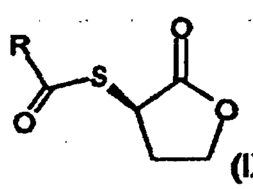

(IXb)

worin R $C_{1-6}$-Alkyl oder Phenyl darstellt, gegebenenfalls substituiert mit einem oder mehreren Substituenten, die aus Halogen, $C_{1-3}$-Alkyl, Hydroxy und $C_{1-3}$-Alkoxy ausgewählt sind; mit einem aliphatischen Alkohol in Gegenwart von Salzsäure.

**9.** Verfahren zur Herstellung von Verbindungen der Formeln (IXa) und (IIIb)

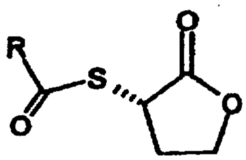

+

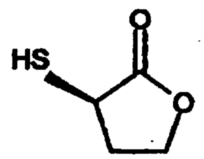

(IXa) (IIIb)

worin R $C_{1-6}$-Alkyl oder Phenyl darstellt, gegebenenfalls substituiert mit einem oder mehren Substituenten, die aus Halogen, $C_{1-3}$-Alkyl, Hydroxy und $C_{1-3}$-Alkoxy ausgewählt sind; umfassend die enzymatische Transacetylierung von racemischer Verbindung (IX):

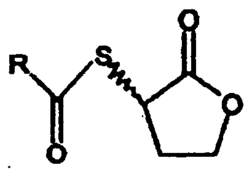

racemisch (IX)

worin R wie oben definiert ist, in Gegenwart eines Alkohols und eines geeigneten Lipase- oder Esterase-Enzyms in einem Lösungsmittel, das Wasser enthält.

**10.** Verfahren zur Herstellung eines einzelnen Enantiomers einer Verbindung der Formel (I):

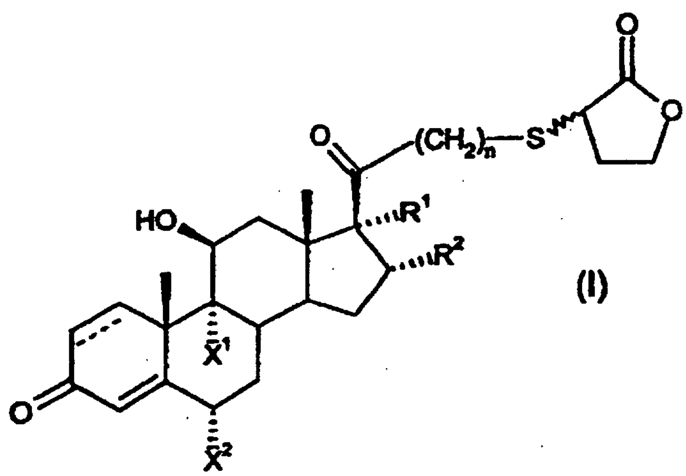

(I)

worin $R^1$ individuell $OC(=)C_{1-6}$-Alkyl darstellt; $R^2$ individuell Wasserstoff, Methyl (das entweder in der α-oder β-Konfiguration sein kann) oder Methylen darstellt; oder $R^1$ und $R^2$ zusammen:

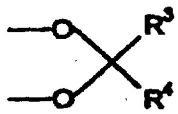

darstellen, worin $R^3$ und $R^4$ gleich oder verschieden sind und jeweils Wasserstoff oder $C_{1-6}$-Alkyl darstellen; $X^1$ und $X^2$ gleich oder verschieden sind und jeweils Wasserstoff oder Halogen darstellen; ------ eine Einfachoder Doppelbindung darstellt; und n = 0 ist; umfassend die Reaktion einer Verbindung der Formel (IIIa) oder (IIIb):

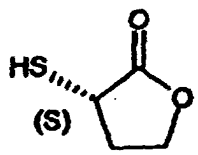

(IIIa)

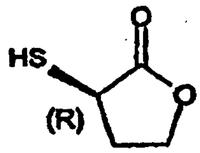

(IIIb)

mit einer Verbindung der Formel (II)

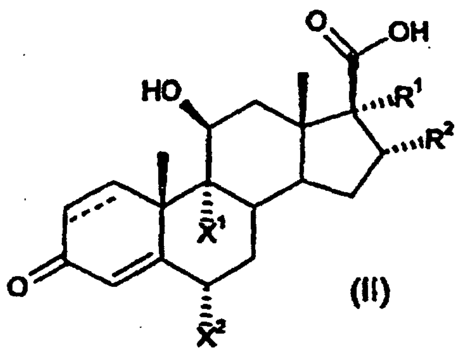

(II)

worin $R^1$, $R^2$, $X^1$ und $X^2$ wie oben für Formel (I) definiert sind, in Gegenwart einer Base und eines Kupplungsmittels.

11. Verfahren gemäß Anspruch 10, worin die Base Natriumbicarbonat ist und das Kupplungsmittel O-Benzotriazol-1-yl-N,N,N',N'-tetramethyluroniumhexafluorophosphat ist.

12. Verfahren zur Herstellung eines einzelnen Enantiomers einer Verbindung der Formel (I):

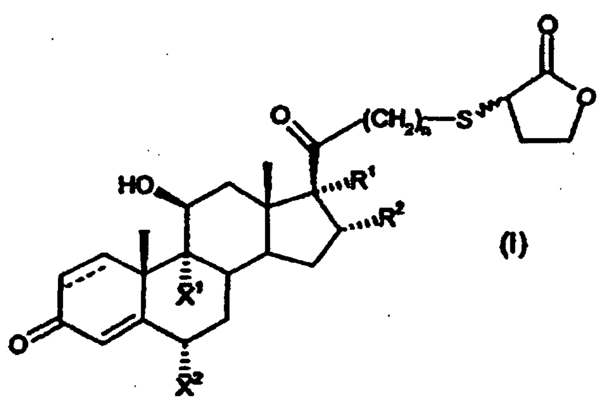

(I)

worin $R^1$ individuell OC(=)$C_{1-6}$-Alkyl darstellt; $R^2$ individuell Wasserstoff, Methyl (das entweder in der α-oder β-Konfiguration sein kann) oder Methylen darstellt; oder $R^1$ und $R^2$ zusammen:

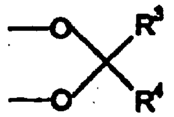

darstellen, worin $R^3$ und $R^4$ gleich oder verschieden sind und jeweils Wasserstoff oder $C_{1-6}$-Alkyl darstellen, $X^1$ und $X^2$ gleich oder verschieden sind und jeweils Wasserstoff oder Halogen darstellen; ------ eine Einfachoder Doppelbindung darstellt; und n = 0 ist; umfassend die Reaktion einer Verbindung der Formel (IIIa) oder (IIIb):

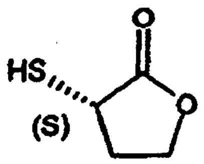

(IIIa)

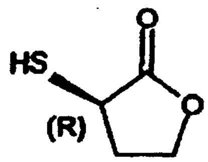

(IIIb)

mit einer Verbindung der Formel (VI)

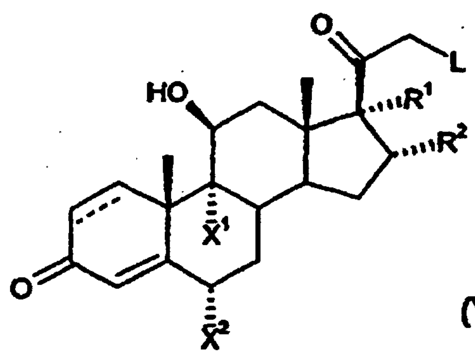

(VI)

worin $R^1$, $R^2$, $X^1$ und $X^2$ wie oben für Formel (I) definiert sind und L eine geeignete Abgangsgruppe darstellt, in Gegenwart einer Base und eines Kupplungsmittels.

**13.** Verfahren gemäß Anspruch 12, worin die Base Natriumbicarbonat ist und das Kupplungsmittel O-Benzotriazol-1-yl-N,N,N',N'-tetramethyluroniumhexafluorophosphat ist.

**Revendications**

**1.** (S)-α-Mercapto-γ-butyrolactone ne contenant pas plus de 10% en poids/poids de l'énantiomère correspondant.

**2.** (S)-α-Mercapto-γ-butyrolactone ne contenant pas plus de 5% en poids/poids de l'énantiomère correspondant.

**3.** (S)-α-Mercapto-γ-butyrolactone contenant moins de 3% en poids/poids de l'énantiomère correspondant.

**4.** (R)-α-Mercapto-γ-butyrolactone ne contenant pas plus de 10% en poids/poids de l'énantiomère correspondant.

**5.** (R)-α-Mercapto-γ-butyrolactone ne contenant pas plus de 5% en poids/poids de l'énantiomère correspondant.

**6.** (R)-α-Mercapto-γ-butyrolactone contenant moins de 3% en poids/poids de l'énantiomère correspondant.

**7.** Procédé pour la préparation d'un composé de formule (IIIa):

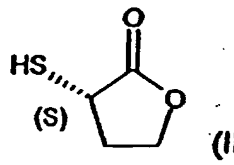

(IIIa)

qui comprend la réaction d'un composé de formule (IXa)

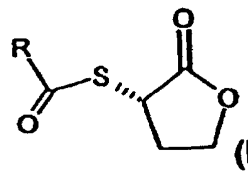

(IXa)

dans laquelle R représente un groupe alkyle en $C_{1-6}$ ou phényle éventuellement substitué par un ou plusieurs substituants choisis parmi un atome d'halogène, un groupe alkyle en $C_{1-3}$, hydroxy et alcoxy en $C_{1-3}$, avec un alcool aliphatique en présence d'acide chlorhydrique.

**8.** Procédé pour la préparation d'un composé de formule (IIIb) :

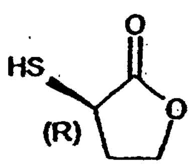

(IIIb)

qui comprend la réaction d'un composé de formule (IXb)

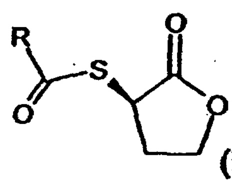

(IXb)

dans laquelle R représente un groupe alkyle en $C_{1-6}$ ou phényle éventuellement substitué par un ou plusieurs substituants choisis parmi un atome d'halogène, un groupe alkyle en $C_{1-3}$, hydroxy et alcoxy en $C_{1-3}$, avec un alcool aliphatique en présence d'acide chlorhydrique.

**9.** Procédé pour la préparation des composés de formules (IXa) et (IIIb) :

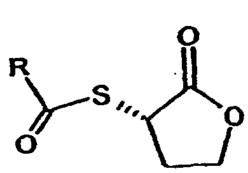

+

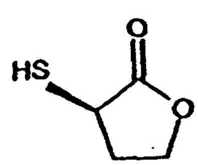

(IXa) . (IIIb)

dans lesquelles R représente un groupe alkyle en $C_{1-6}$ ou phényle éventuellement substitué par un ou plusieurs

substituants choisis parmi un atome d'halogène, un groupe alkyle en $C_{1-3}$, hydroxy et alcoxy en $C_{1-3}$, qui comprend la transacétylation enzymatique du composé racémique (IX) :

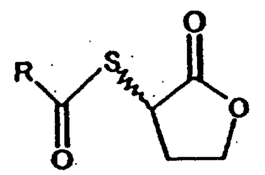

(IX) racémique

dans laquelle R est tel que défini plus haut, en présence d'un alcool et d'une enzyme lipase ou estérase dans un solvant contenant de l'eau.

**10.** Procédé pour la préparation d'un énantiomère individuel d'un composé de formule (I) :

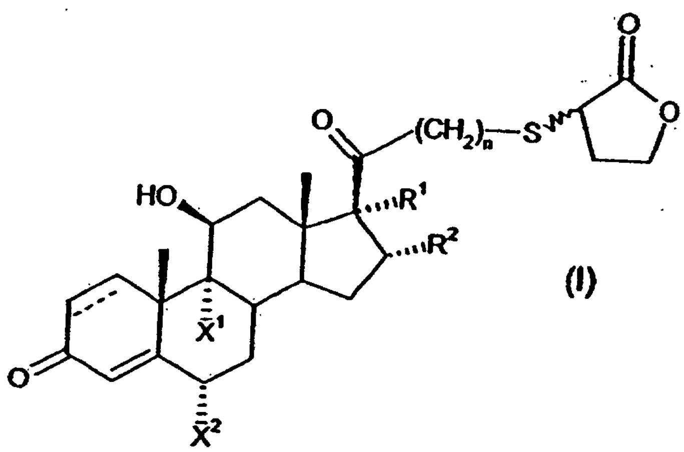

(I)

dans laquelle $R^1$ représente individuellement un groupe OC(=O)-alkyle en $C_{1-6}$ ; $R^2$ représente individuellement un atome d'hydrogène, un groupe méthyle (qui peut être en configuration $\alpha$ ou $\beta$) ou méthylène ; ou bien $R^1$ et $R^2$ forment ensemble :

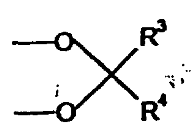

où $R^3$ et $R^4$ sont identiques ou différents et représentent chacun un atome d'hydrogène ou un groupe alkyle en $C_{1-6}$ ; $X^1$ et $X^2$ sont identiques ou différents et représentent chacun un atome d'hydrogène ou d'halogène ; $\overline{----}$ représente une liaison simple ou double ; et n=0 ; qui comprend la réaction d'un composé de formule (IIIa) ou (IIIb) :

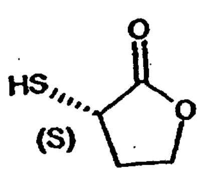

(IIIa)

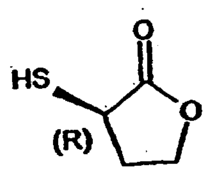

(IIIb)

avec un composé de formule (II) :

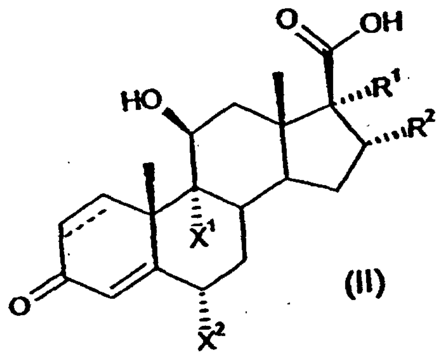

(II)

dans laquelle $R^1$, $R^2$, $X^1$ et $X^2$ sont tels que définis plus haut pour la formule (I), en présence d'une base et d'un agent de couplage.

**11.** Procédé selon la revendication 10, dans lequel la base est l'hydrogénocarbonate de sodium et l'agent de couplage est l'hexafluorophosphate de O-benzotriazole-1-yl-N,N,N',N'-tétraméthyluronium.

**12.** Procédé pour la préparation d'un énantiomère individuel d'un composé de formule (I) :

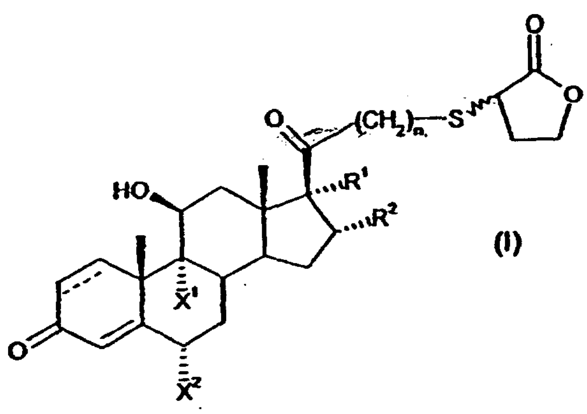

(I)

dans laquelle $R^1$ représente individuellement un groupe OC(=O)-alkyle en $C_{1-6}$ ; $R^2$ représente individuellement un atome d'hydrogène, un groupe méthyle (qui peut être en configuration α ou β) ou méthylène ; ou bien $R^1$ et $R^2$ forment ensemble :

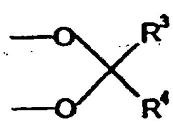

où $R^3$ et $R^4$ sont identiques ou différents et représentent chacun un atome d'hydrogène ou un groupe alkyle en $C_{1-6}$ ; $X^1$ et $X^2$ sont identiques ou différents et représentent chacun un atome d'hydrogène ou d'halogène ; ---- représente une liaison simple ou double ; et n=1 ; qui comprend la réaction d'un composé de formule (IIIa) ou (IIIb) :

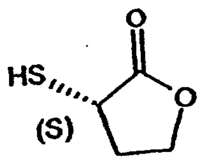 (IIIa) 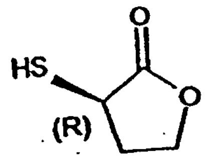 (IIIb)

avec un composé de formule (VI) :

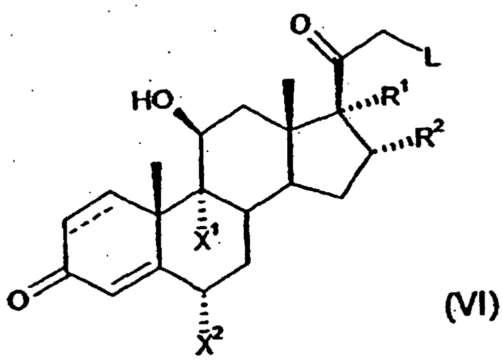

(VI)

dans laquelle $R^1$, $R^2$, $X^1$ et $X^2$ sont tels que définis plus haut pour la formule (I) et L représente un groupe partant convenable, en présence d'une base et d'un agent de couplage.

**13.** Procédé selon la revendication 12, dans lequel la base est l'hydrogénocarbonate de sodium et l'agent de couplage est l'hexafluorophosphate de O-benzotriazole-1-yl-N,N,N',N'-tétraméthyluronium.